# EUROPEAN PATENT SPECIFICATION

(11) **EP 3 087 973 B1**
(45) Date of publication and mention of the grant of the patent: **05.02.2020**
(21) Application number: 13900130.9
(22) Date of filing: 23.12.2013
(51) Int. Cl.: A61K 9/127, A61K 31/203, A61K 8/67, A61Q 19/08, A61Q 19/00, A61K 8/14, A61K 9/08, A61K 31/11

(54) **METHOD FOR PREPARING LIPOSOMES OF RETINALDEHYDE OR OTHER PRECURSORS OF RETINOIC ACID AND PRODUCT THUS OBTAINED**
VERFAHREN ZUR HERSTELLUNG VON LIPOSOMEN AUS RETINALDEHYD ODER ANDEREN VORLÄUFERN VON RETINOESÄURE UND IN DIESEM VERFAHREN ERHALTENES PRODUKT
PROCÉDÉ DE PRÉPARATION DE LIPOSOMES DE RÉTINALDÉHYDE OU D'AUTRES PRÉCURSEURS DE L'ACIDE RÉTINOÏQUE, ET PRODUIT AINSI OBTENU

(43) Date of publication of application: 02.11.2016
(73) Proprietor: Dermopartners, S.L., 46138 Rafelbunyol - Valencia (ES)
(72) Inventor: SERRANO SANMIGUEL, Gabriel, 46138 Rafelbunyol - Valencia (ES); SERRANO NUÑEZ, Juan Manuel, 46138 Rafelbunyol - Valencia (ES); NAVARRO MOLINER, Maria, 46138 Rafelbunyol - Valencia (ES)
(74) Representative: Urizar Anasagasti, Jesus Maria
(86) International application number: PCT/ES2013/070922
(87) International publication number: WO 2015/097317

(56) References cited:
- WO-A1-98/14167
- WO-A2-02/32413
- WO-A2-2007/115134
- US-A- 4 911 928
- YU-KYOUNG OH ET AL: "Skin permeation of retinol in Tween 20-based deformable liposomes: in-vitro evaluation in human skin and keratinocyte models", JOURNAL OF PHARMACY AND PHARMACOLOGY, vol. 58, no. 2, 1 February 2006 (2006-02-01), pages 161-166, XP055379216, LONDON; GB ISSN: 0022-3573, DOI: 10.1211/jpp.58.2.0002
- SCHAFER-KORTING ET AL.: 'Lipid nanoparticles for improved topical application of drugs for skin diseases' ADVANCED DRUG DELIVERY REVIEWS vol. 59, no. 6, 10 July 2007, AMSTERDAM, NL, pages 427 - 443, XP022205270
- GONNET M ET AL.: 'New trends in encapsulation of liposoluble vitamins' JOURNAL OF CONTROLLED RELEASE vol. 146, no. 3, 15 September 2010, AMSTERDAM, NL, pages 276 - 290, XP055292689

## Description

### OBJECT OF THE INVENTION

The invention that is proposed relates to a new method for preparing liposomes of retinaldehyde and the resulting product which has an application in the sector of beauty, cosmetic and dermatology centres.

### BACKGROUND OF THE INVENTION

To date, there are different preparations known to contain retinaldehyde as an active agent in their composition. Until now, preparations with concentrations of 1 % retinal were not used due to the intense yellow colour and the difficulty to solubilise it in any kind of pharmaceutical form.

Some preparations are made with non-phospholipidic liposomes, some with liposomes with cholesterol and others with lamellar emulsions. This is the case, for example, for patents US2003118616 and WO9631194. It's also known the patent WO9814167 referred to a cosmetic compositions in which the homogenization of an aqueous and an oily phase is described but nothing is said about obtaining liposomes.

The invention herein described relates to the method for the obtention and to the use of phospholipid liposomes that, while preventing degradation to retinal or protecting another precursor of retinoic acid from external agents they preserve their efficacy over a long period of time. Liposomes have a lipid bilayer similar to that of the stratum corneum, which confers a good affinity with the skin, favouring its penetration and allowing to work with lower concentrations of the active agent.

This way, in addition to protecting the active agent, the same level of effectiveness as that of higher concentrations is achieved and also the irritation caused by the retinoids applied topically is prevented.

### DESCRIPTION OF THE INVENTION

The procedure to obtain the product and the product obtained as proposed by the invention consists in a method for obtaining a suspension of phospholipid liposomes with retinaldehyde.

To produce the suspension of liposomes of retinaldehyde, firstly the active agent, retinaldehyde is solubilised in a solvent compatible with the lipids. Polyoxyethylene 20 sorbitan monolaurate is added to this mixture. The concentration of lecithin may vary from 46 mg/mL to 184 mg/mL. If polyoxyethylene 20 sorbitan monolaurate is used, the quantity used varies from 12.5 mg/mL to 50 mg/mL.

The mixture will be added to an aqueous phase, distilled water or saline in the reaction equipment and it will be homogenised for 30 minutes. This process takes place at a temperature below 35°C and is carried out under vacuum. The obtained product can be used directly or mixed at a ratio of 3:1 or 1:1 with distilled water or saline and a preservative agent.

The resulting suspension is filtered through a filter of 0.2 microns. This process results in phospholipid liposomes of retinaldehyde that are sized between 100 and 150 nm and with concentrations of retinaldehyde at a ratio of 0.01 % to 1 % in an appropriate pharmaceutical form.

### PREFERRED EMBODIMENT OF THE INVENTION

As the preferred embodiment of the invention, the method for obtaining the solution of liposomes of retinaldehyde or another precursor of retinoic acid is carried out in the following way:
- The active agent, retinal is solubilised in a compatible solvent along with the lipids.
- The polyoxyethylene 20 sorbitan monolaurate is added to this mixture. The concentration of lecithin may vary from 46 mg/mL to 184 mg/mL If polyoxyethylene 20 sorbitan monolaurate is used, the quantity used varies from 12.5 mg/mL to 50 mg/mL.
- The mixture will be added to an aqueous phase, distilled water or saline in the reaction equipment and it will be homogenised for 30 minutes. This process takes place at a temperature below 35° C and is carried out under vacuum.
- The obtained product can be used directly or mixed at a ratio of 3:1 or 1:1 with distilled water or saline and a preservative agent.
- The resulting solution is filtered through a filter of 0.2 microns.

The result is a solution that allows preparing cosmetic or dermatological products with concentrations of retinaldehyde at a ratio of 0.01 % to 1 %.

Having sufficiently described the nature of the present invention, it must be noted that this invention may undergo variations in its components and percentages, provided that said variations do not substantially vary the characteristics that are claimed in the following section:

## Claims

1. A method for preparing liposomes of retinaldehyde that contain, at least partially retinaldehyde as an active agent, at a concentration of 0.01 % to 1 % **characterised in that** it comprises the following steps:
- The active agent, retinaldehyde is solubilised in a compatible solvent along with the lipids where the concentration of lecithin of the lipids may vary from 46 mg/mL to 184 mg/mL.
- The polyoxyethylene 20 sorbitan monolaurate in a concentration from 12.5 mg/mL to 50 mg/mL is added to this mixture.
- The mixture is added to an aqueous phase, distilled water or saline in the reaction equipment and is homogenised for 30 minutes at a temperature below 35°C and under vacuum.
- The product obtained can be used directly or mixed at a ratio of 3:1 or 1:1 with distilled water or saline and a preservative agent.
- The resulting solution is filtered through a filter of 0.2 microns.

2. Liposomes of retinaldehyde obtained in the way indicated in claim 1 which are phospholipidic in nature and **characterised in that** the liposomes contain, at least partially, retinaldehyde as an active agent, at a concentration of 0.01 % to 1 %.

## Patentansprüche

1. Verfahren zur Herstellung von Retinaldehydliposomen, die mindestens teilweise Retinaldehyd als Wirkstoff enthalten, in einer Konzentration von 0,01 % bis 1 %, **dadurch gekennzeichnet, dass** es die folgenden Schritte umfasst:
- Der Wirkstoff Retinaldehyd wird zusammen mit den Lipiden in einem kompatiblen Lösungsmittel solubilisiert, wobei die Lecithinkonzentration der Lipide von 46 mg/ml bis 184 mg/ml variieren kann.
- Zu dieser Mischung wird das Polyoxyethylen-20-sorbitanmonolaurat in einer Konzentration von 12,5 mg/ml bis 50 mg/ml gegeben.
- Die Mischung wird zu einer wässrigen Phase, destilliertem Wasser oder Kochsalzlösung in der Reaktionsapparatur gegeben und 30 Minuten bei einer Temperatur unter 35 °C und unter Vakuum homogenisiert.
- Das erhaltene Produkt kann direkt verwendet oder in einem Verhältnis von 3:1 oder 1:1 mit destilliertem Wasser oder Kochsalzlösung und einem Konservierungsmittel gemischt werden.
- Die resultierende Lösung wird durch einen Filter von 0,2 Mikrometer filtriert.

2. Retinaldehydliposomen, die auf die in Anspruch 1 angegebene Weise erhalten werden, phospholipidischer Natur sind und **dadurch gekennzeichnet sind, dass** die Liposomen mindestens teilweise Retinaldehyd als Wirkstoff in einer Konzentration von 0,01 % bis 1 % enthalten.

## Revendications

1. Procédé de préparation de liposomes de rétinaldéhyde qui contiennent, au moins partiellement du rétinaldéhyde en tant qu'agent actif, à une concentration de 0,01 % à 1 % **caractérisé en ce qu'**il comprend les étapes suivantes :
- L'agent actif, le rétinaldéhyde est solubilisé dans un solvant compatible avec les lipides où la concentration en lécithine des lipides peut varier de 46 mg/ml à 184 mg/ml.
- Le monolaurate polyoxyéthylène 20 sorbitane à une concentration de 12,5 mg/ml à 50 mg/ml est ajouté à ce mélange.
- Le mélange est ajouté à une phase aqueuse, de l'eau distillée ou une solution saline dans l'équipement de réaction et est homogénéisé pendant 30 minutes à une température en dessous de 35 °C et sous vide.
- Le produit obtenu peut être utilisé directement ou mélangé dans un rapport de 3:1 ou 1:1 avec de l'eau distillée ou une solution saline et un agent conservateur.
- La solution résultante est filtrée à travers un filtre de 0,2 micron.

2. Liposomes de rétinaldéhyde obtenus de la manière indiquée dans la revendication 1 qui sont de nature phospholipidique et **caractérisés en ce que** les liposomes contiennent, au moins partiellement, du rétinaldéhyde en tant qu'agent actif, à une concentration de 0,01 % à 1 %.
